# FASCICULE DE BREVET EUROPEEN

(11) **EP 1 707 180 B1**
(45) Date de publication et mention de la délivrance du brevet: **04.05.2016**
(21) Numéro de dépôt: 06290509.6
(22) Date de dépôt: 31.03.2006
(51) Int. Cl.: A61Q 5/04, A61K 8/97

(54) **Compositions cosmétiques ou dermatologiques, comprenant du disulfure de sélénium, une base lavante et au moins un éther à deux chaînes grasses, particulier, et procédés de traitement cosmétique**
Kosmetische oder dermatologische Zusammensetzungen enthaltend Selendisulfid, eine waschaktive Base und wenigstens einen Ether mit zwei bestimmten Fettsäureketten und Verfahren zur kosmetischen Behandlung
Cosmetic or dermatological compositions comprising selendioxide, a surfactant and an ether with at least two specific fatty chains and processes for cosmetic treatment

(30) Priorité: 31.03.2005 FR 0503153; 31.03.2005 FR 0503154
(43) Date de publication de la demande: 04.10.2006
(73) Titulaire: L'Oréal, 75008 Paris (FR)
(72) Inventeur: Decoster, Sandrine, 95210 Saint-Gratien (FR); Meralli, Sabrina, 92170 Vanves (FR)
(74) Mandataire: Dossmann, Gérard

(56) Documents cités:
- EP-A- 0 524 859
- EP-A- 0 717 981
- EP-A- 0 976 393
- EP-A- 1 457 194
- EP-A1- 1 088 546
- WO-A-02/30370
- WO-A-99/09944
- FR-A- 1 393 607
- US-A- 4 854 333

## Description

La présente invention est relative à des compositions cosmétiques ou dermatologiques, comprenant du disulfure de sélénium une base lavante, et événtuellement au moins un éther à deux chaînes grasses particulier, et à des procédés de traitement cosmétique des cheveux mettant en oeuvre lesdites compositions cosmétiques.

En vue de combattre la formation des pellicules qui est généralement accompagnée d'une prolifération microbienne et/ou fongique, il a été proposé comme produits anti-pelliculaires soit des produits inhibant la prolifération microbienne, soit des produits kératolytiques. Parmi ces derniers, l'emploi du disulfure de sélénium a été tout particulièrement préconisé en raison de sa puissante activité cytostatique (voir "Science des traitements capillaires, Ch. Zviak, 1988, page 98).

Cependant, si le disulfure de sélénium possède une excellente activité anti-pelliculaire, il présente néanmoins l'inconvénient de brunir progressivement dans le temps passant de l'orangé au brun-vert.

Afin de remédier à ce problème de changement de couleur, le document US 4 854 333 a proposé d'associer au disulfure de sélénium un agent oxydant de type peroxyde ou persel. Toutefois, l'emploi d'agents oxydants peut présenter des problèmes de toxicité et/ou de compatibilité dans les compositions.

Une autre solution au problème de changement de couleur a été proposée dans EP 717 981. Elle consiste à associer au disulfure de sélénium un sel de zinc d'un acide minéral ou organo-carboxylique pour obtenir des compositions présentant une couleur stable au cours du temps.

Cependant, la présence de sels de zinc peut être préjudiciable à l'ajustement de la viscosité des compositions et au dépôt des agents conditionneurs éventuellement présents dans les compositions.

Par composition de "couleur stable" on entend au sens de la présente invention, une composition qui, conservée pendant au moins un mois dans une étuve à 45 °C, ne présente pas de modification sensible de couleur par rapport à sa couleur initiale.

En outre, le disulfure de sélénium présente l'inconvénient de conduire à des formulations de shampoing présentant des qualités d'usage médiocres, et notamment une difficulté d'étalement sur les cheveux et le cuir chevelu, pouvant entraîner une efficacité moyenne du traitement anti-pelliculaire. Il est également peu stable dans les compositions.

Afin de remédier au problème de stabilité dans des formulations de shampoing, le document US 4 885 107 a proposé d'associer du disulfure de sélénium à un alcanolamide et à un agent de mise en suspension de type dérivé à longue chaîne ou gomme de xanthane. Le dérivé à longue chaîne peut être un diester d'éthylèneglycol, un alcanolamide d'acide gras, un ester à longue chaîne d'acide gras, un ester de glycéryle ou un oxyde d'alkyl (C₁₆₋₂₂) diméthylamine.

Afin de surmonter les inconvénients de l'art antérieur, la demanderesse a maintenant découvert de manière surprenante qu'en formulant du disulfure de sélénium, dans des compositions présentant un profil rhéologique particulier caractérisé par un temps de relaxation de 1 à 18 ms., mesuré à 25 °C, il est possible d'obtenir une meilleure répartition sur les cheveux et le cuir chevelu que les produits disponibles sur le marché, tels que ceux vendus sous les marques commerciales Dercos^{®} et Head and Shoulders^{®}, et donc une meilleure efficacité en ce qui concerne le traitement anti-pelliculaire.

En outre, cette association conduit à de bonnes qualités d'usage telles qu'une mousse abondante et aérée, un développement rapide de la mousse, et une facilité et rapidité de rinçage, ainsi qu'à de bonnes propriétés cosmétiques sur les cheveux, telles que douceur et lissage.

De plus, l'association du disulfure de sélénium à un éther à deux chaînes grasses particulier permet également d'obtenir une composition de couleur stable pendant son stockage au cours du temps.

Cette stabilité de la couleur peut encore être améliorée par l'adjonction d'au moins un alcool gras comportant au moins 18 atomes de carbone.

Un autre avantage des compositions selon l'invention consiste en ce que l'on peut ajuster aisément leur viscosité à l'aide d'épaississants classiques.

Les propriétés cosmétiques obtenues avec les compositions selon l'invention sont également bonnes.

La présente invention a donc pour objet une composition cosmétique ou dermatologique comprenant, dans un milieu aqueux, une base lavante et du disulfure de sélénium, et présentant un profil rhéologique défini par un temps de relaxation de 1 à 18 ms, mesuré à 25 °C.

Un autre objet est une composition cosmétique ou dermatologique, comprenant dans un milieu aqueux, du disulfure de sélénium, une base lavante et au moins un éther à deux chaînes grasses tel que décrit ci-dessous.

La présente invention a encore pour objet une utilisation d'au moins un éther à deux chaînes grasses et d'au moins un alcool gras comportant au moins 18 atomes de carbone dans une composition contenant du disulfure de sélénium pour obtenir un profil rhéologique défini par un temps de relaxation de 1 à 18 ms., mesuré à 25 °C.

D'autres objets de l'invention sont des procédés de traitement cosmétique mettant en oeuvre lesdites compositions.

D'autres objets et caractéristiques, aspects et avantages de l'invention apparaîtront encore plus clairement à la lecture de la description et des exemples qui suivent.

Selon l'invention, la composition cosmétique ou dermatologique, comprend dans un milieu aqueux, une base lavante et du disulfure de sélénium, et présentant un profil rhéologique défini par un temps de relaxation de 1 à 18 ms, mesuré à 25 °C.

Le temps de relaxation correspond à l'inverse de la fréquence du point de croisement entre le module élastique (G') et le module visqueux (G").

Il peut être mesuré à l'aide d'un rhéomètre rotatif Thermo RS600, équipé d'un corps de géométrie cône-plan. La température a été maintenue à 25 °C.

De préférence, la composition selon l'invention comprend au moins un éther à deux chaînes grasses tel que décrit ci-dessous.

Un autre objet de l'invention est une composition cosmétique ou dermatologique, comprenant dans un milieu aqueux :
- du disulfure de sélénium,
- au moins un éther à deux chaînes grasses tel que décrit ci-dessous, et
- une base lavante.

Ces compositions, notamment sous forme de shampooing, ainsi obtenues, présentent alors de bonnes qualités d'usage, à savoir une mousse abondante et aérée, un développement rapide de la mousse, et une facilité et rapidité de rinçage, ainsi que de très bonnes propriétés cosmétiques sur les cheveux, telles que douceur et lissage.

Le disulfure de sélénium utilisé dans la présente invention comporte essentiellement un atome de sélénium et deux atomes de soufre. Il peut également se présenter sous la forme d'une structure de polysulfure SeₓS_{y} dans laquelle x+y = 8.

Le disulfure de sélénium se présente sous la forme d'une poudre dont les particules ont une granulométrie inférieure à 200 µm, de préférence inférieure à 25 µm.

Le disulfure de sélénium est de préférence présent dans les compositions de l'invention en une proportion allant de 0,001 à 10 % en poids, mieux encore de 0,1 à 5 % en poids, et encore plus préférentiellement de 0,2 à 2 % en poids par rapport au poids total de la composition.

Les éthers à deux chaînes grasses utilisés dans la présente invention, sont choisis parmi les éthers à deux chaînes grasses, solides à une température égale à environ 30°C, répondant à la formule suivante :

R-O-R' (I)

dans laquelle :
R et R', identiques, désignent un groupe alkyle, linéaire ou ramifié, comportant de 14 à 24 atomes de carbone, R et R' étant choisis de façon telle que le composé de formule (I) soit solide à une température égale à 30°C environ.

De préférence, R et R' désignent un groupe alkyle tel que stéaryle.

Les éthers à deux chaînes grasses utilisables selon l'invention sont insolubles dans les compositions cosmétiques ou dermatologiques, et peuvent être préparés selon le procédé décrit dans la demande de brevet DE 41 27 230.

On utilise notamment dans le cadre de la présente invention, le distéaryléther vendu sous la dénomination CUTINA^{®} STE par la société COGNIS.

L'éther à deux chaînes grasses est présent de préférence en une proportion supérieure ou égale à 0,5 % en poids, mieux encore de 1 à 5 % en poids, et encore plus préférentiellement de 1,3 à 2 % en poids, par rapport au poids total de la composition.

La base lavante est constituée d'au moins un tensioactif choisi parmi les tensioactifs anioniques, non-ioniques et amphotères ou zwittérioniques tels que décrits ci-dessous.

Les tensioactifs anioniques pouvant être utilisés dans la composition sont notamment choisis parmi les sels, en particulier les sels de métaux alcalins tels que les sels de sodium, les sels d'ammonium, les sels d'amines, les sels d'aminoalcoals ou les sels de métaux alcalino-ferreux, par exemple, de magnésium, des types suivants : les alkylsulfates, les alkyléthersulfates, les alkylamidoéther-sulfates, les alkylarylpolyéthersulfates, les monoglycéride-sulfates ; les alkylsulfonates, les alkylamidesulfonates, les alkylarylsulfonates, les α-oléfine-sulfonates, les paraffine-sulfonates, les alkyl-sulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

Lorsqu'ils sont présents, la quantité du ou des tensioactifs anioniques est de préférence comprise dans l'intervalle allant de 2,5 à 50 % en poids, mieux encore de 4 à 20% en poids par rapport au poids total de la composition.

Les tensioactifs non-ioniques utilisables dans les compositions de la présente invention sont des composés bien connus en soi (voir notamment à cet égard "Handbook of Surfactants" par M.R. PORTER, éditions Blackie & Son (Glasgow and London), 1991, pp 116-178). Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitane éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Les agents tensioactifs amphotères ou zwittérioniques, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3^{ème} édition, 1982, sous les dénominations Amphocarboxyglycinate et Amphocarboxypropionate de structures respectives (II) et (III) :

Rₐ-CONHCH₂CH₂-N(R_{b})(R_{c})(CH₂COO) (II)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ; et

   Rₐ'-CONHCH₂CH₂-N(B)(B') (III)

   dans laquelle :
   B représente -CH₂CH₂OX',
   B' représente -(CH₂)_{z}-Y', avec z = 1 ou 2,
   X' représente le groupe -CH₂CH₂-COOH ou un atome d'hydrogène,
   Y' représente -COOH ou le groupe -CH₂-CHOH-SO₃H,
   Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTF_{A}, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Lorsqu'ils sont présents, la quantité du ou des tensioactifs amphotères ou zwittérioniques est de préférence comprise dans l'intervalle allant de 0,2 à 20 % en poids, mieux encore de 0,5 à 10% en poids par rapport au poids total de la composition.

Un mode de réalisation particulier consiste en ce que la base lavante est constituée d'au moins un tensioactif anionique, d'au moins un tensioactif non-ionique et d'au moins un tensioactif amphotère ou zwittérionique.

De manière avantageuse, la base lavante est constituée d'au moins un tensioactif anionique et d'au moins un tensioactif amphotère ou zwittérionique.

Une autre base lavante particulière est constituée d'au moins un tensioactif anionique et d'au moins un tensioactif non-ionique.

La quantité de base lavante est de préférence comprise dans l'intervalle allant de 4 à 50 % en poids, mieux encore de 4 à 20 % en poids par rapport au poids total de la composition.

Les compositions selon l'invention peuvent comprendre en outre au moins un alcool gras comportant au moins 18 atomes de carbone, de préférence de 18 à 30 atomes de carbone et encore plus préférentiellement de 18 à 24 atomes de carbone.

A titre d'exemples de tels alcools gras, on peut notamment citer les alcools stéarylique, arachidylique, béhénylique, lignocérylique, cérylique et montanylique, et leurs mélanges, et plus particulièrement l'alcool béhénylique.

L'alcool gras est présent de préférence en une proportion supérieure ou égale à 0,5 % en poids, mieux encore de 1 à 5 % en poids, et encore plus préférentiellement de 1,3 à 2 % en poids, par rapport au poids total de la composition.

Les compositions selon la présente invention peuvent comprendre en outre au moins un tensioactif cationique.

A titre d'exemples de tensioactif cationique, on peut notamment citer les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique.

Lorsque les tensioactifs cationiques sont présents, leur quantité est de préférence comprise dans l'intervalle allant de 0,1 à 10 % en poids, mieux encore de 0,2 à 5 % en poids, et encore plus préférentiellement de 0,3 à 3 % en poids par rapport au poids total de la composition cosmétique.

Les compositions selon la présente invention peuvent comprendre en outre au moins un polymère cationique.

Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont une masse moléculaire moyenne en poids supérieure à 10⁵, de préférence supérieure à 10⁶ et mieux encore comprise entre 10⁶ et 10⁸.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont ceux décrits dans les brevets français n⁵ 2 505 348 et 2 542 997. Parmi ces polymères, on peut citer :
(1) les homopolymères ou copolymères dérivés d'esters ou d'amides acryliques ou méthacryliques et comportant au moins un des motifs de formules suivantes: 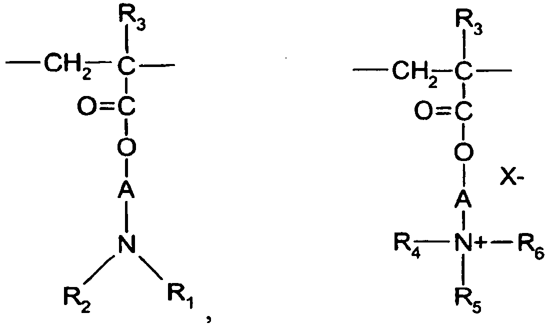 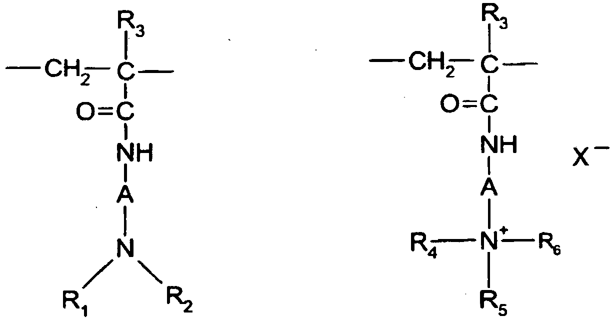 dans lesquelles:
   R₁ et R₂, identiques ou différents, représentent un atome d'hydrogène ou un groupe alkyle ayant de 1 à 6 atomes de carbone, et de préférence un groupe méthyle ou éthyle ;
   R₃, identiques ou différents, désignent un atome d'hydrogène ou un groupe CH₃ ;
   les symboles A, identiques ou différents, représentent un groupe alkyle, linéaire ou ramifié, comportant de 1 à 6 atomes de carbone, de préférence 2 ou 3 atomes de carbone, ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle, et de préférence un groupe alkyle ayant de 1 à 6 atomes de carbone ;
   X désigne un anion dérivé d'un acide minéral ou organique tel qu'un anion méthosulfate ou un halogénure comme le chlorure ou le bromure.

   Les copolymères de la famille (1) peuvent contenir en outre un ou plusieurs motifs dérivant de comonomères pouvant être choisis dans la famille des acrylamides, méthacrylamides, diacétone-acrylamides, acrylamides et méthacrylamides substitués sur l'atome d'azote par des groupes alkyle inférieur (C₁-C₄), des groupes dérivés des acides acryliques ou méthacryliques ou de leurs esters, de vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisé au sulfate de diméthyle ou avec un halogénure de diméthyle,
   - les copolymères d'acrylamide et de chlorure de méthacryloyloxyéthyltriméthylammonium décrits, par exemple, dans la demande de brevet EP-A-080976,
   - les copolymères d'acrylamide et de méthosulfate de méthacryloyioxyéthyltriméthyl ammonium,
   - les copolymères vinylpyrrolidone/acrylate ou méthacrylate de dialkylaminoalkyle quaternisés ou non. Ces polymères sont décrits en détail dans les brevets français 2 077 143 et 2 393 573,
   - les terpolymères méthacrylate de diméthylaminoéthyle/ vinylcaprolactame/vinylpyrrolidone,
   - les copolymères vinylpyrrolidone/méthacrylamidopropyl-diméthylamine, et
   - les copolymères vinylpyrrolidone/méthacrylamide de diméthylaminopropyle quaternisé.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium, de diméthyldiallylammonium.
   Les produits commercialisés répondant à cette définition sont plus particulièrement les produits vendus sous la dénomination "Celquat^{®} L 200" et "Celquat^{®} H 100" par la Société National Starch. (4) Les polysaccharides cationiques décrits dans les brevets US 3 589 578 et 4 031 307 tels que les gommes de guar contenant des groupements cationiques trialkylammonium. On utilise, par exemple, des gommes de guar modifiées par un sel, par exemple le chlorure, de 2,3-époxypropyltriméthylammonium.
   De tels produits sont commercialisés notamment sous les dénominations commerciales de JAGUAR^{®} C 13 S, JAGUAR^{®} C15, JAGUAR^{®} C17 ou JAGUAR^{®} C162 par la société MEYHALL.
(5) Les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361.
(6) Les polyaminoamides solubles dans l'eau, préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alkylés ou s'ils comportent une ou plusieurs fonctions amines tertiaires, quaternisées. De tels polymères sont notamment décrits dans les brevets français 2 252 840 et 2 368 508.
(7) Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyldialkylènetriamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.
   Parmi ces dérivés, on peut citer plus particulièrement les polymères acide adipique/diméthylaminohydroxypropyl-diéthylènetriamine.
(8) Les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.
(9) Les cyclopolymères d'alkyldiallylamine ou de dialkyldiallylammonium tels que les homopolymères ou copolymères comportant, comme constituant principal de la chaîne, des motifs répondant aux formules (Va) ou (Vb) : 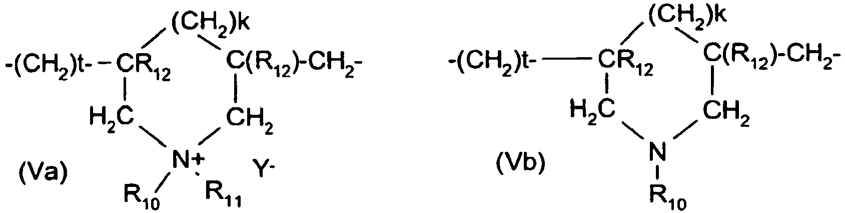 dans lesquelles k et t sont égaux à 0 ou 1, la somme k + t étant égale à 1 ; R₁₂ désigne un atome d'hydrogène ou un groupe méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou alors R₁₀ et R₁₁ peuvent désigner conjointement avec l'atome d'azote auquel ils sont rattachés, des groupements hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "MERQUAT^{®} 100" par la société CALGON (et ses homologues de faibles masses moléculaires moyenne en poids) et les copolymères de chlorure de diallyldiméthylammonium et d'acrylamide commercialisés sous la dénomination "MERQUAT^{®} 550".
(10) Les polymères de diammonium quaternaire contenant des motifs récurrents répondant à la formule (VI) : 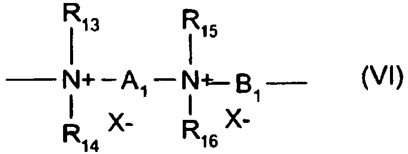 dans laquelle :
   R₁₃, R₁₄, R₁₅ et R₁₆, identiques ou différents, représentent des groupes aliphatiques, alicycliques ou arylaliphatiques contenant de 1 à 20 atomes de carbone ou des groupes hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁-C₆, linéaire ou ramifié, substitué par un groupement nitrile, ester, acyle, amide ou -CO-O-R₁₇-E ou -CO-NH-R₁₇-E où R₁₇ est un groupe alkylène et E un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre, si A1 désigne un groupe alkylène ou hydroxyalkylène linéaire ou ramifié, saturé ou insaturé, B₁ peut également désigner un groupement :

      -(CH₂)ₙ-CO-E'-OC-(CH₂)ₙ-

      dans lequel E' désigne :
      a) un reste de glycol de formule -O-Z-O-, où Z désigne un groupe hydrocarboné linéaire ou ramifié, ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ;
      d) un groupement uréylène de formule -NH-CO-NH- .

   De préférence, X⁻ est un anion tel que le chlorure ou le bromure.
   Des polymères de ce type sont notamment décrits dans les brevets français 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.
   On peut utiliser plus particulièrement les polymères qui sont constitués de motifs récurrents répondant à la formule : 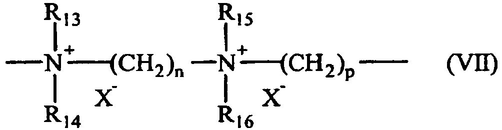 dans laquelle R₁₃, R₁₄, R₁₅ et R₁₆, identiques ou différents, désignent un groupe alkyle ou hydroxyalkyle ayant de 1 à 4 atomes de carbone environ, n et p sont des nombres entiers variant de 2 à 20 environ et, X⁻ est un anion dérivé d'un acide minéral ou organique.
(11) Les polymères de polyammonium quaternaire constitués de motifs de formule (VIII) : 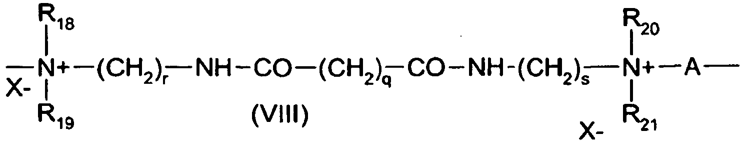 dans laquelle :
   R₁₈, R₁₉, R₂₀ et R₂₁, identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH, où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X- désigne un anion tel qu'un halogénure,
   A désigne un radical d'un dihalogénure ou représente de préférence -CH₂-CH₂-O-CH₂-CH₂-.

   De tels composés sont notamment décrits dans la demande de brevet EP-A-122 324.
(12) Les polymères quaternaires de vinylpyrrolidone et de vinylimidazole.
(13) Les polymères réticulés de sels de méthacryloyloxyalkyl(C₁-C₄) trialkyl(C₁-C₄)ammonium tels que les polymères obtenus par homopolymérisation du méthacrylate de diméthylaminoéthyle quaternisé par le chlorure de méthyle, ou par copolymérisation de l'acrylamide avec le méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bisacrylamide.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose comportant des groupements ammonium quaternaires tels que les produits vendus sous la dénomination "JR 400" par la Société UNION CARBIDE CORPORATION, les cyclopolymères cationiques, en particulier les homopolymères ou copolymères de chlorure de diméthyldiallylammonium, vendus sous les dénominations MERQUAT^{®} 100, MERQUAT^{®} 550 et MERQUAT^{®} S par la société CALGON, les gommes de guar modifiées par un sel de 2,3-époxypropyltriméthylammonium, les polymères quaternaires de vinylpyrrolidone et de vinylimidazole.

Lorsque les polymères cationiques sont présents, ils sont de préférence présents en une quantité allant de 0,01 à 10 % en poids, mieux encore de 0,02 à 5 % en poids, et encore plus préférentiellement de 0,05 à 1 % en poids par rapport au poids total de la composition.

Les compositions selon l'invention peuvent comprendre en outre au moins une silicone.

Les silicones utilisables conformément à l'invention peuvent être solubles ou insolubles dans la composition. Elles peuvent être en particulier des polyorganosiloxanes insolubles dans la composition de l'invention et se présenter sous forme d'huiles, de cires, de résines ou de gommes.

Les silicones insolubles sont notamment dispersées dans les compositions sous forme de particules ayant généralement une taille moyenne en nombre comprise entre 2 nanomètres et 100 micromètres, de préférence entre 20 nanomètres et 20 micromètres (mesurée avec un granulomètre).

Les organopolysiloxanes sont définis plus en détail dans l'ouvrage de Walter NOLL "Chemistry and Technology of Silicones" (1968) Academic Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 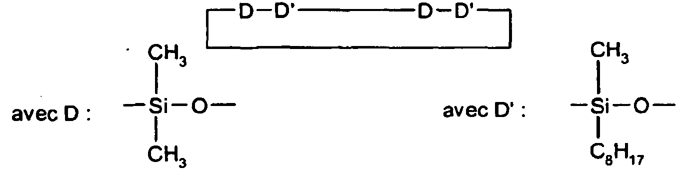 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels, les copolymères blocs linéaires polysiloxane(A)-polyoxyalkylène(B) de type (A-B)ₙ avec n >3 ; les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; ainsi que leurs mélanges.

A titre d'exemples de polyalkylsiloxanes, on peut notamment citer les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

Dans cette classe de polyalkylsiloxanes, on peut également citer les produits commercialisés sous les dénominations "ABIL WAX 9800 et 9801 par la société GOLDSCHMIDT qui sont des polyalkyl (C₁-C₂₀) siloxanes.

Les polyalkylarylsiloxanes peuvent être notamment choisis parmi les polydiméthyl-méthylphénylsiloxanes, les polydiméthyl-diphényl-siloxanes linéaires et/ou ramifiés de viscosité de 1.10⁻⁵ à 5.10⁻² m²/s à 25°C.

Parmi ces polyalkylarylsiloxanes, on peut citer à titre d'exemple les produits commercialisés sous les dénominations suivantes :
- les huiles SILBIONE de la série 70 641 de RHONE POULENC ;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane,
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des silicones que l'on peut utiliser dans la composition selon l'invention sont des mélanges tels que :
. les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 20 et 8.5 % d'une huile SF 96.

Les résines d'organopolysiloxanes utilisables conformément à l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle.

Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements ammonium quaternaires comme les produits commercialisés sous les dénominations ABILQUAT 3272 et ABILQUAT 3474 par la société GOLDSCHMIDT ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201" et "ABIL^{®} S255".
- des groupements hydroxyacylamino, comme les polyorganosiloxanes décrits dans la demande EP 342 834. On peut citer, par exemple, le produit Q2-8413 de la société DOW CORNING.

Les silicones particulièrement préférées dans l'invention sont les polydiméthylsiloxanes tels que les polydiméthylsiloxanes à groupements terminaux triméthylsilyle, ou les polydiméthylsiloxanes à groupements terminaux hydroxydiméthylsilyle, et les silicones aminées.

Lorsque lesdites silicones présentes, elles sont contenues de préférence en une quantité allant de 0,05 à 20 % en poids, plus particulièrement de 0,1 à 10 % en poids, et mieux encore de 0,5 à 5 % en poids par rapport au poids total de la composition.

Le milieu aqueux est constitué d'eau ou d'un mélange d'eau et d'au moins un solvant cosmétiquement ou dermatologiquement acceptable choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

Le pH des compositions selon l'invention est généralement inférieur à 8,5, et de préférence compris dans l'intervalle allant de 4 à 7.

La composition selon l'invention peut comprendre en outre un ou plusieurs additifs classiques bien connus dans la technique, tels que les agents antipelliculaires différents du disulfure de sélénium, les agents antichute, les agents oxydants, les céramides et pseudo-céramides, les vitamines et pro-vitamines dont le panthénol, les huiles végétales, animales, minérales et synthétiques, les cires, les filtres solaires, les pigments minéraux et organiques, colorés ou non colorés, les colorants, les agents nacrants et opacifiants, les agents séquestrants, les agents plastifiants, les agents solubilisants, les agents acidifiants, des agents alcalinisants, les agents épaississants minéraux et organiques, les agents anti-oxydants, les hydroxyacides, les parfums et les agents conservateurs.

L'homme de métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

Un autre objet de l'invention est l'utilisation d'au moins un éther à deux chaînes grasses et d'au moins un alcool gras comportant au moins 18 atomes de carbone tels que définis ci-dessus, dans une composition contenant du disulfure de sélénium pour obtenir un profil rhéologique défini par un temps de relaxation de 1 à 18 ms., mesuré à 25 °C.

Cette utilisation permet d'obtenir aussi de bonnes qualités d'usage telles que mousse abondante et aérée, développement rapide de la mousse, et facilité et rapidité de rinçage, lors de l'application de la composition sur les cheveux et le cuir chevelu, ainsi que de bonnes propriétés cosmétiques telles que douceur et lissage, après application de la composition.

Un autre objet de l'invention est un procédé de traitement cosmétique des cheveux, qui consiste à appliquer une quantité efficace d'une composition cosmétique telle que décrite ci-dessus, sur lesdites matières, à rincer après un éventuel temps de pause.

Les exemples suivants sont donnés à titre illustratif de la présente invention.

Dans les exemples suivants, toutes les quantités sont indiquées en pour cent en poids de matière active par rapport au poids total de la composition, sauf indication contraire.

### EXEMPLES

### Exemple 1

On prépare la composition suivante à partir des ingrédients indiqués dans le tableau ci-dessous.

| | |
|---|---|
| Lauryl éther sulfate de sodium | 14,7 % |
| Cocoylbétaïne | 2,4 % |
| Disulfure de sélénium | 1 % |
| Distéaryléther | 1,5 % |
| Mélange d'alcools gras vendu sous la marque commerciale NAFOL 1822C par la société SASOL | 1,5 % |
| Polymère carboxyvinylique (Carbopol^{®} 980) | 0,4 % |
| Polydiméthylsiloxane (Dow Corning 200 Fluid 60 000cSt) | 2 % |
| Parfum | 0,5 % |
| Acide citrique qs | pH 4,8-5,2 |
| Eau qsp | 100 % |

On obtient un gel nacré orangé.

Au bout de deux mois de conservation à 45 °C, on n'a observé aucune évolution de la couleur et l'activité anti-pelliculaire est conservée.

Cette composition est un shampooing que l'on applique sur cheveux humides en massant le cuir chevelu. On la laisse reposer deux minutes et on rince.

Après séchage, les cheveux sont doux et déliés. On observe également une bonne efficacité antipelliculaire.

On a effectué les mesures rhéologiques à l'aide d'un rhéomètre rotatif Thermo RS600, équipé d'une géométrie cône-plan, 60 mm/1° (titane). La température a été maintenue à 25 °C.

Les résultats obtenus sont regroupés dans le tableau ci-dessous.

| | Temps de relaxation (ms) |
|---|---|
| Composition A | 11 |
| Produit Dercos^{®} au disulfure de sélénium⁽¹⁾ | 20 |
| Produit H&S^{®} au disulfure de sélénium⁽²⁾ | 32 |

| | |
|---|---|
| ⁽¹⁾ : comprenant de l'alcool cétylique et de l'hydroxystéaryl cétyléther. ⁽²⁾ : comprenant du distéarate de glycol. | |

Les produits Dercos^{®} et H&S^{®} présentent un temps de relaxation en dehors de la gamme de l'invention.

En outre, la composition A présente de meilleures qualités d'usage que les produits Dercos^{®} et H&S^{®}.

### Exemple 2

On prépare la composition suivante à partir des ingrédients indiqués dans le tableau ci-dessous.

| | |
|---|---|
| Lauryl sulfate de sodium | 14,7 % |
| Disodium cocoamphodiacétate | 3,5 % |
| Disulfure de sélénium | 0,8 % |
| Distéaryléther | 2 % |
| Mélange d'alcools gras vendu sous la marque commerciale NAFOL 1822C par la société SASOL | 2 % |
| Polymère carboxyvinylique (Carbopol^{®} 980) | 0,4 % |
| Polydiméthylsiloxane (Dow Corning 200 Fluid 60 000cSt) | 2 % |
| Parfum | qs |
| Acide citrique qs | pH 4,8-5,2 |
| Eau qsp | 100 % |

### Exemple 3

On prépare la composition suivante à partir des ingrédients indiqués dans le tableau ci-dessous.

| | |
|---|---|
| Lauryl éther sulfate de sodium à 2,2 moles d'oxyde d'éthylène (ou OE) | 10 % |
| Acide lauryl éther carboxylique à 4.5 moles d'OE | 10 % |
| Disulfure de sélénium | 1,5 % |
| Distéaryléther | 1,5 % |
| Mélange d'alcools gras vendu sous la marque commerciale NAFOL 1822C par la société SASOL | 0,9 % |
| Polyvinylpyrrolidone | 0,02 % |
| Polydiméthylsiloxane (Dow Corning 200 Fluid 60 000cSt) | 0,5 % |
| Parfum | qs |
| Acide citrique qs | pH 4,8-5,2 |
| Eau qsp | 100 % |

### Exemple 4

On prépare la composition suivante à partir des ingrédients indiqués dans le tableau ci-dessous.

| | |
|---|---|
| Lauryl sulfate de sodium | 8 % |
| Lauryl éther sulfate de sodium à 2,2 moles d'oxyde d'éthylène (ou OE) | 10 % |
| Cocoamidopropylbétaine | 2,5 % |
| Laurate de glycéryle | 0,5 % |
| Disulfure de sélénium | 0,5 % |
| Distéaryléther | 1,5 % |
| Mélange d'alcools gras vendu sous la marque commerciale NAFOL 1822C par la société SASOL | 1,5 % |
| Polymère cationique Jaguar C13S | 0,02 % |
| Polymère carboxyvinylique (Carbopol^{®} 980) | 0,4 % |
| Polydiméthylsiloxane (Dow Corning 200 Fluid 60 000cSt) | 2 % |
| Parfum | qs |
| Acide citrique qs | pH 4,8-5,2 |
| Eau qsp | 100 % |

## Revendications

1. Composition cosmétique ou dermatologique comprenant, dans un milieu aqueux,
- du disulfure de sélénium,
- au moins un éther à deux chaînes grasses choisi parmi les éthers à deux chaînes grasses, solides à une température égale à environ 30°C, répondant à la formule suivante :
R-O-R' (I)
dans laquelle :
R et R', identiques, désignent un groupe alkyle linéaire ou ramifié, comportant de 14 à 24 atomes de carbone, R et R' étant choisis de façon telle que le composé de formule (I) soit solide à une température égale à 30° C environ, et
- une base lavante.

2. Composition selon la revendication 1, **caractérisée en ce que** l'éther à deux chaînes grasses est le distéaryléther.

3. Composition selon la revendication 1 ou 2, **caractérisée en ce que** l'éther à deux chaînes grasses est présent en une quantité supérieure ou égale à 0,5 % en poids par rapport au poids total de la composition.

4. Composition selon la revendication 3, **caractérisée en ce que** l'éther à deux chaînes grasses est contenu en une quantité allant de 1 à 5 % en poids, de préférence de 1,3 à 2 % en poids, par rapport au poids total de la composition.

5. Composition selon l'une quelconque des revendications précédentes, **caractérisée en ce que** la base lavante est constituée d'au moins un tensioactif choisi parmi les tensioactifs anioniques, non ioniques, amphotères ou zwittérioniques.

6. Composition selon la revendication 5, **caractérisée en ce que** la base lavante est constituée d'au moins un tensioactif anionique et d'au moins un tensioactif amphotère ou zwittérionique, ou d'au moins un tensioactif anionique et d'au moins un tensioactif non ionique, ou d'au moins un tensioactif anionique, d'au moins un tensioactif non ionique et d'au moins un tensioactif amphotère ou zwittérionique.

7. Composition selon la revendication 5 ou 6, **caractérisée en ce que** le tensioactif anionique est choisi parmi les alkylsulfates, les alkyléthersulfates et leurs mélanges.

8. Composition selon l'une quelconque des revendications 5 à 7, **caractérisée en ce que** le tensioactif amphotère ou zwittérionique est choisi parmi les (alkyl en C₈₋₂₀)-bétaïnes, les (alkyl en C₈₋₂₀)-amido(alkyl en C₆₋₈)bétaïnes et leurs mélanges.

9. Composition selon l'une quelconque des revendications précédentes, **caractérisée en ce que** la base lavante est présente en une quantité allant de 4 à 50 % en poids, de préférence de 4 à 20 % en poids par rapport au poids total de la composition.

10. Composition selon l'une quelconque des revendications précédentes, **caractérisée en ce qu'**elle comprend en outre au moins un alcool gras comportant au moins 18 atomes de carbone.

11. Composition selon la revendication 10, **caractérisée en ce que** l'alcool gras comporte de 18 à 30 atomes de carbone.

12. Composition selon la revendication 10 ou 11, **caractérisée en ce que** l'alcool gras est choisi parmi les alcools stéarylique, arachidylique, béhénylique, lignocérylique, cérylique et montanylique, et leurs mélanges.

13. Composition selon l'une quelconque des revendications 10 à 12, **caractérisée en ce que** l'alcool gras est présent en une quantité supérieure ou égale à 0,5 % en poids par rapport au poids total de la composition.

14. Composition selon la revendication 13, **caractérisée en ce que** l'alcool est présent en une quantité allant de 1 à 5 % en poids, de préférence de 1,3 à 2 % en poids, par rapport au poids total de la composition.

15. Composition selon l'une quelconque des revendications précédentes, **caractérisée en ce qu'**elle comprend en outre au moins un polymère cationique.

16. Composition selon l'une quelconque des revendications précédentes, **caractérisée en ce qu'**elle comprend en outre au moins une silicone.

17. Composition selon l'une quelconque des revendications précédentes, **caractérisée en ce que** le milieu aqueux est constitué d'eau ou d'un mélange d'eau et d'au moins un solvant cosmétiquement ou dermatologiquement acceptable.

18. Composition selon la revendication 17, **caractérisée en ce que** le solvant cosmétiquement ou dermatologiquement acceptable est choisi parmi les alcools inférieurs en C₁-C₄ et les polyols.

19. Composition selon l'une quelconque des revendications précédentes, **caractérisée en ce qu'**elle comprend en outre au moins un additif choisi parmi les agents antipelliculaires différents du disulfure de sélénium, les agents antichute, les agents oxydants, les céramides et pseudo-céramides, les vitamines et pro-vitamines dont le panthénol, les huiles végétales, animales, minérales et synthétiques, les cires, les filtres solaires, les pigments minéraux et organiques, colorés ou non colorés, les colorants, les agents nacrants et opacifiants, les agents séquestrants, les agents plastifiants, les agents solubilisants, les agents acidifiants, des agents alcalinisants, les agents épaississants minéraux et organiques, les agents anti-oxydants, les hydroxyacides, les parfums et les agents conservateurs.

20. Utilisation de la composition selon l'une quelconque des revendications précédentes, comme shampooing.

21. Utilisation d'une composition selon l'une quelconque des revendications 10 à 19, pour obtenir des qualités d'usage lors de l'application de la composition sur les cheveux et le cuir chevelu, qui sont une mousse abondante et aérée, un développement rapide de la mousse, et une facilité et rapidité de rinçage.

22. Utilisation d'une composition selon l'une quelconque des revendications 10 à 19, pour obtenir des propriétés cosmétiques après application de la composition, qui sont la douceur et le lissage des cheveux.

23. Procédé de traitement cosmétique, comprenant l'application d'une quantité efficace d'une composition cosmétique selon l'une quelconque des revendications 1 à 19, sur les cheveux.

## Patentansprüche

1. Kosmetische oder dermatologische Zusammensetzung, die in einem wässrigen Medium
- Selendisulfid,
- mindestens einen Ether mit zwei Fettketten, ausgewählt aus Ethern mit zwei Fettketten, die bei einer Temperatur von ungefähr 30°C fest sind und der folgenden Formel entsprechen:
R-O-R' (I)
in der:
R und R' gleich sind und für eine lineare oder verzweigte Alkylgruppe mit 14 bis 24 Kohlenstoffatomen stehen, wobei R und R' so gewählt sind, dass die Verbindung der Formel (I) bei einer Temperatur von ungefähr 30°C fest ist, und
- eine Waschbasis
umfasst.

2. Zusammensetzung nach Anspruch 1, **dadurch gekennzeichnet, dass** es sich bei dem Ether mit zwei Fettketten um Distearylether handelt.

3. Zusammensetzung nach Anspruch 1 oder 2, **dadurch gekennzeichnet, dass** der Ether mit zwei Fettketten in einer Menge größer gleich 0,5 Gew.-%, bezogen auf das Gesamtgewicht der Zusammensetzung, vorliegt.

4. Zusammensetzung nach Anspruch 3, **dadurch gekennzeichnet, dass** der Ether mit zwei Fettketten in einer Menge im Bereich von 1 bis 5 Gew.-%, vorzugsweise von 1,3 bis 2 Gew.-%, bezogen auf das Gesamtgewicht der Zusammensetzung, enthalten ist.

5. Zusammensetzung nach einem der vorhergehenden Ansprüche, **dadurch gekennzeichnet, dass** die Waschbasis aus mindestens einem Tensid, das aus anionischen, nichtionischen, amphoteren oder zwitterionischen Tensiden ausgewählt ist, besteht.

6. Zusammensetzung nach Anspruch 5, **dadurch gekennzeichnet, dass** die Waschbasis aus mindestens einem anionischen Tensid und mindestens einem amphoteren oder zwitterionischen Tensid oder mindestens einem anionischen Tensid und mindestens einem nichtionischen Tensid oder mindestens einem anionischen Tensid, mindestens einem nichtionischen Tensid und mindestens einem amphoteren oder zwitterionischen Tensid besteht.

7. Zusammensetzung nach Anspruch 5 oder 6, **dadurch gekennzeichnet, dass** das anionische Tensid aus Alkylsulfaten, Alkylethersulfaten und Mischungen davon ausgewählt ist.

8. Zusammensetzung nach einem der Ansprüche 5 bis 7, **dadurch gekennzeichnet, dass** das amphotere oder zwitterionische Tensid aus (C₈₋₂₀-Alkyl)betainen, (C₈₋₂₀-Alkyl)amido (C₆₋₈-alkyl)betainen und Mischungen davon ausgewählt ist.

9. Zusammensetzung nach einem der vorhergehenden Ansprüche, **dadurch gekennzeichnet, dass** die Waschbasis in einer Menge im Bereich von 4 bis 50 Gew.-%, vorzugsweise 4 bis 20 Gew.-%, bezogen auf das Gesamtgewicht der Zusammensetzung, vorliegt.

10. Zusammensetzung nach einem der vorhergehenden Ansprüche, **dadurch gekennzeichnet, dass** sie außerdem mindestens einen Fettalkohol mit mindestens 18 Kohlenstoffatomen umfasst.

11. Zusammensetzung nach Anspruch 10, **dadurch gekennzeichnet, dass** der Fettalkohol 18 bis 30 Kohlenstoffatome enthält.

12. Zusammensetzung nach Anspruch 10 oder 11, **dadurch gekennzeichnet, dass** der Fettalkohol aus Stearylalkohol, Arachidylalkohol, Behenylalkohol, Lignocerylalkohol, Cerylalkohol und Montanylalkohol und Mischungen davon ausgewählt ist.

13. Zusammensetzung nach einem der Ansprüche 10 bis 12, **dadurch gekennzeichnet, dass** der Fettalkohol in einer Menge größer gleich 0,5 Gew.-%, bezogen auf das Gesamtgewicht der Zusammensetzung, vorliegt.

14. Zusammensetzung nach Anspruch 13, **dadurch gekennzeichnet, dass** der Alkohol in einer Menge im Bereich von 1 bis 5 Gew.-%, vorzugsweise von 1,3 bis 2 Gew.-%, bezogen auf das Gesamtgewicht der Zusammensetzung, vorliegt.

15. Zusammensetzung nach einem der vorhergehenden Ansprüche, **dadurch gekennzeichnet, dass** sie außerdem mindestens ein kationisches Polymer umfasst.

16. Zusammensetzung nach einem der vorhergehenden Ansprüche, **dadurch gekennzeichnet, dass** sie außerdem mindestens ein Silikon umfasst.

17. Zusammensetzung nach einem der vorhergehenden Ansprüche, **dadurch gekennzeichnet, dass** das wässrige Medium aus Wasser oder einer Mischung von Wasser und mindestens einem kosmetisch bzw. dermatologisch unbedenklichen Lösungsmittel besteht.

18. Zusammensetzung nach Anspruch 17, **dadurch gekennzeichnet, dass** das kosmetisch bzw. dermatologisch unbedenkliche Lösungsmittel aus C₁-C₄-Niederalkoholen und Polyolen ausgewählt ist.

19. Zusammensetzung nach einem der vorhergehenden Ansprüche, **dadurch gekennzeichnet, dass** sie außerdem mindestens ein Additiv umfasst, das aus Antischuppenmitteln, die von Selendisulfid verschieden sind, Mitteln zur Bekämpfung von Haarverlust, Oxidationsmitteln, Ceramiden und Pseudoceramiden, Vitaminen und Provitaminen, darunter Panthenol, pflanzlichen, tierischen, mineralischen und synthetischen Ölen, Wachsen, Lichtschutzmitteln, farbigen oder farblosen anorganischen und organischen Pigmenten, Farbmitteln, Glanz- und Trübungsmitteln, Sequestriermitteln, Weichmachern, Lösungsvermittlern, Ansäuerungsmitteln, Alkalinisierungsmitteln, anorganischen oder organischen Verdickungsmitteln, Antioxidantien, Hydroxysäuren, Parfümen und Konservierungsmitteln ausgewählt ist.

20. Verwendung der Zusammensetzung nach einem der vorhergehenden Ansprüche als Shampoo.

21. Verwendung einer Zusammensetzung nach einem der Ansprüche 10 bis 19 zum Erhalt von Verwendungsqualitäten beim Aufbringen der Zusammensetzung auf das Haar und die Kopfhaut, bei denen es sich um einen üppigen und luftigen Schaum, eine schnelle Schaumentwicklung und leichtes und schnelles Ausspülen handelt.

22. Verwendung einer Zusammensetzung nach einem der Ansprüche 10 bis 19 zum Erhalt von kosmetischen Eigenschaften nach dem Aufbringen der Zusammensetzung, bei denen es sich um Weichheit und Glätte des Haars handelt.

23. Verfahren zur kosmetischen Behandlung, bei dem man eine wirksame Menge einer kosmetischen Zusammensetzung nach einem der Ansprüche 1 bis 19 auf das Haar aufbringt.

## Claims

1. Cosmetic or dermatological composition comprising, in an aqueous medium,
- selenium disulfide,
- at least one ether with two fatty chains, chosen from ethers with two fatty chains which are solid at a temperature equal to approximately 30°C, corresponding to the following formula:
R-O-R' (I)
in which:
R and R' , which are identical, denote a linear or branched alkyl group comprising from 14 to 24 carbon atoms, R and R' being chosen such that the compound of formula (I) is solid at a temperature equal to approximately 30°C, and
- a washing base.

2. Composition according to Claim 1, **characterized in that** the ether with two fatty chains is distearyl ether.

3. Composition according to Claim 1 or 2, **characterized in that** the ether with two fatty chains is present in an amount of greater than or equal to 0.5% by weight, relative to the total weight of the composition.

4. Composition according to Claim 3, **characterized in that** the ether with two fatty chains is contained in an amount ranging from 1 to 5% by weight, preferably from 1.3 to 2% by weight, relative to the total weight of the composition.

5. Composition according to any one of the preceding claims, **characterized in that** the washing base is constituted of at least one surfactant chosen from anionic, nonionic, amphoteric or zwitterionic surfactants.

6. Composition according to Claim 5, **characterized in that** the washing base is constituted of at least one anionic surfactant and at least one amphoteric or zwitterionic surfactant, or of at least one anionic surfactant and at least one nonionic surfactant, or of at least one anionic surfactant, at least one nonionic surfactant and at least one amphoteric or zwitterionic surfactant.

7. Composition according to Claim 5 or 6, **characterized in that** the anionic surfactant is chosen from alkyl sulfates, alkyl ether sulfates and mixtures thereof.

8. Composition according to any one of Claims 5 to 7, **characterized in that** the amphoteric or zwitterionic surfactant is chosen from (C₈₋₂₀ alkyl)betaines, (C₈₋₂₀ alkyl)amido(C₆₋₈ alkyl)betaines and mixtures thereof.

9. Composition according to any one of the preceding claims, **characterized in that** the washing base is present in an amount ranging from 4 to 50% by weight, preferably from 4 to 20% by weight, relative to the total weight of the composition.

10. Composition according to any one of the preceding claims, **characterized in that** it also comprises at least one fatty alcohol comprising at least 18 carbon atoms.

11. Composition according to Claim 10, **characterized in that** the fatty alcohol comprises from 18 to 30 carbon atoms.

12. Composition according to Claim 10 or 11, **characterized in that** the fatty alcohol is chosen from stearyl, arachidyl, behenyl, lignoceryl, ceryl and montanyl alcohols, and mixtures thereof.

13. Composition according to any one of Claims 10 to 12, **characterized in that** the fatty alcohol is present in an amount of greater than or equal to 0.5% by weight, relative to the total weight of the composition.

14. Composition according to Claim 13, **characterized in that** the alcohol is present in an amount ranging from 1 to 5% by weight, preferably from 1.3 to 2% by weight, relative to the total weight of the composition.

15. Composition according to any one of the preceding claims, **characterized in that** it also comprises at least one cationic polymer.

16. Composition according to any one of the preceding claims, **characterized in that** it also comprises at least one silicone.

17. Composition according to any one of the preceding claims, **characterized in that** the aqueous medium is constituted of water or of a mixture of water and at least one cosmetically or dermatologically acceptable solvent.

18. Composition according to Claim 17, **characterized in that** the cosmetically or dermatologically acceptable solvent is chosen from C₁-C₄ lower alcohols and polyols.

19. Composition according to any one of the preceding claims, **characterized in that** it also comprises at least one additive chosen from anti-dandruff agents other than selenium disulfide, agents for combating hair loss, oxidizing agents, ceramides and pseudoceramides, vitamins and provitamins including panthenol, vegetable, animal, mineral and synthetic oils, waxes, sunscreens, coloured or colourless inorganic and organic pigments, dyes, pearlescent agents and opacifiers, sequestrants, plasticizers, solubilizers, acidifying agents, basifying agents, inorganic and organic thickeners, antioxidants, hydroxy acids, fragrances and preservatives.

20. Use of the composition according to any one of the preceding claims, as shampoo.

21. Use of a composition according to any one of Claims 10 to 19, to obtain use qualities, during application of the composition to the hair and the scalp, of a copious and aerated mousse, rapid development of the mousse, and easy and rapid rinsing.

22. Use of a composition according to any one of Claims 10 to 19, to obtain cosmetic properties, after application of the composition, of softness and smoothing of the hair.

23. Cosmetic treatment method, comprising the application to the hair of an effective amount of a cosmetic composition according to any one of Claims 1 to 19.
